(19) Europäisches Patentamt / European Patent Office / Office européen des brevets

(11) **EP 4 324 489 A1**

(12) **EUROPEAN PATENT APPLICATION**

(43) Date of publication:
**21.02.2024  Bulletin 2024/08**

(21) Application number: **22191338.7**

(22) Date of filing: **19.08.2022**

(51) International Patent Classification (IPC):
*A61L 27/04* (2006.01)        *A61L 31/02* (2006.01)
*B21C 23/00* (2006.01)        *C22C 18/00* (2006.01)
*B22F 1/052* (2022.01)        *B22F 3/04* (2006.01)
*B22F 3/20* (2006.01)          *C22C 1/04* (2023.01)
*C22C 1/05* (2023.01)          *C22C 1/10* (2023.01)
*C22C 32/00* (2006.01)

(52) Cooperative Patent Classification (CPC):
**A61L 27/047; A61L 31/022; A61L 31/148;
B21C 23/007; B22F 1/052; B22F 3/20;
C22C 1/0483; C22C 1/10; C22C 18/00;
C22C 32/0042;** B22F 2003/247; C22C 1/0441;
C22C 1/1094

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Centrum pre vyuzitie pokrocilych materialov
  Slovenskej akademie vied, verejna vyskumna
  institucia
  845 11 Bratislava - mestska cast Karlova Ves (SK)**

• **Ustav materialov a mechaniky strojov Slovenskej
  akademie vied, verejna vyskumna instiucia
  845 13 Bratislava - mestska cast Karlova Ves (SK)**

(72) Inventors:
• **Balog, Martin
  841 07 Bratislava (SK)**
• **Krizik, Peter
  902 03 Pezinok (SK)**

(74) Representative: **Majlingová, Zuzana
  Majlingová & Partners, s.r.o.
  Budatínska 12
  851 06 Bratislava (SK)**

(54) **A BIOCOMPATIBLE AND BIOABSORBABLE COMPOSITE MATERIAL FOR FULL ABSORPTION IN VIVO IN CONTACT WITH A HUMAN OR ANIMAL TISSUE AND METHOD OF MANUFACTURE OF SAID COMPOSITE MATERIAL**

(57)    A biocompatible and bioabsorbable composite material for full absorption *in vivo* in contact with a human or animal tissue, characterized in that, it comprises: a bioabsorbable zinc or zinc-alloy matrix forming a fine-grained (50 nm - 10 μm) bearing structure; and 0.1 to 10 % vol. of a bioabsorbable component, relative to the volume of the composite material, absorbable *in vivo* in contact with a human or animal tissue, in the form of particles dispersed substantially homogenously in the matrix, wherein particle size is 10 nm to 5 μm, preferably below 0.5 μm.

**Description**

**Technical filed**

The invention relates to bioabsorable zinc-based composite materials for biomedical applications.

**Background art**

Coronary artery disease (CAD) is the foremost cause of death worldwide, creating a major economic burden on patients' quality of life and public health systems [1]. Common way to treat CAD is stenting by traditional inert metals (stainless steel or cobalt-chromium alloys). Inert endovascular stents (ES) stay in the human body for many years and causes long-term health risks such as impaired coronary vasomotion, development of early neoatherosclerosis, late-stage thrombosis, chronic inflammation, etc. [2]. Furthermore, the surgical implementation of internal fixation metallic implants (wires, plates, screws, staples) is commonly applied in orthopedics for the purpose of repairing a bone. After the time, which is necessary to support a recovering tissue, orthopedic internal fixators (OIF) become redundant; its long-term retention becomes problematic e.g., the refracture rate increases and has to be removed. However, a secondary operation is most stressful to a patient, and brings economical burdens.

Owing to their unique combination of properties metals represent traditionally an appealing option for bioabsorbable implant applications in particular low profile OIF and ES with a thin strut section. Among other bioabsorbable metals, namely magnesium (Mg) and iron (Fe), Zinc (Zn) has attracted considerable increase in scientific interest recently as it addresses some of the major issues related with using of Mg and Fe biometals [3].

Zn, which is considered as a nutrition essential trace element in the human body, plays structural, regulatory, and catalytic roles in the human cell [4]. Zn resorbs at the rate, which falls between: i) the difficult to control high corrosion rate of Mg and ii) the degradation rate of Fe, which is too slow [3]. The intermediate corrosion rate of Zn provides an engineering advantage to design a low profile ES (with a requested penetration rate ($<20\ \mu m \cdot y^{-1}$) and OIF ($0.5\ \mu m \cdot y^{-1}$), which retain mech. integrity and full absorption for required times of 3-6 months and 1-2 y, respectively [3, 5]. Owing to a low melting point ($T_m$) and low reactivity, Zn can be processed easily and simply by conventional techniques of casting and wrought processing.

However, bioabsorbable Zn materials are not without shortcomings. Most importantly pure Zn shows rather low mechanical performance compared to Fe and Mg, which may jeopardize mechanical integrity namely of low-profile ES [3].

Alloying of Zn with elements, which are biocompatible or essential to human function, is the most logical solution applied by many, which increases the poor strength of pure Zn [3]. Another feasible approach is cold/warm working of coarse-grained Zn structures by e.g, drawing, direct hydroextrusion (HE) or conventional extrusion (CE) operations at high strains and low strain rates resulting in refined, equiaxed (sub)grain structure, which assures a significant strength increase accompanied with reasonably high average strain rate ($\varepsilon$) [3].

But for all of these developed Zn alloys some major problems remain unresolved. It was shown that refined ZnMg structures are prone to post-deformation instability of mechanical properties, so-called "annealing strengthening", which limits application potential of Zn biometals [6]. Herein, the significant strengths increase and severe drop of $\varepsilon$ were observed after post-deformation lagering (even short term), due to the natural age hardening i.e., overaging related to grain structure regeneration and $Zn_{11}Mg_2$ precipitation processes. Expectedly, near and submicrometre Zn grain structures tend to creep profoundly at the temperature of a human body, yet the creep results on bioabsorbable Zn alloys are scarce [7].

Besides, an increased induced strain gave a rise of structural inhomogeneity i.e., an existence of shear bands with pronounced tendency to DRX, and flow localization, which in turn affected workability [8]. Furthermore, some works reported Zn biometals being prone to the strain softening, while it may have a negative impact on uniformity of expansion and recoil of ES [6]. The mechanical properties of Zn are strongly strain rate sensitive and steeply deteriorate with an increase of temperature, what bears important implications on the processability and applicability [6].

US 5254/0353835 A1 discloses a biomedical device that includes a Zn-based matrix and nanostructures dispersed in the matrix and a method of its production. It discloses a broad family of bioresorbable Zn based metal matrix composites with fine grained Zn grain structure (4-12 $\mu m$) stiffened, stabilized and strengthened by nanocomponent ($<1\ \mu m$) of a broad range of ceramic particles (carbides, oxides, etc.) with the content >2.5vol.% fabricated by combination of solid state and molten route mostly for biomedical applications.

However, there are several disadvantages of the disclosed device and method. Firstly, there is a limitation in a minimal grain size, which can be attained by the process. Major improvement in terms of strength, fatigue and creep endurance takes place for ultrafine-grained structures i.e., for the grain size $<1\ \mu m$. Furthermore, ceramic reinforcement increases Young's modulus of the composite, whereas Young's modulus shall rather decrease in order to avoid the problems related to a stress shielding effect. The ex-situ fabrication process poses a high risk of reinforcement clustering and minimal reinforcement's size to be accommodated successfully, with an expected negative effect on performance and toxicity. Other risk related to process carried out at elevated temperatures is formation of unwanted products due to reaction at matrix-reinforcement interface. Other limitation is that some enclosed reinforcement systems are not biocompatible and may induce a toxic effect by various means upon Zn matrix resorption.

The aim of this invention is to provide a Zinc-based bioabsorbable composite material capable of dissolving fully and safely in the body at a moderate absorption rate, with improved mechanical properties.

**Summary of the invention**

It has been surprisingly found out that the mechanical properties of the bioabsorable Zn-based composite material are considerably improved if the composite material comprises a small fraction of certain nanoscale components (dispersoids), such as zinc oxide, hydroxyapatite, zinc hydrogen phosphate, that are homogenously dispersed in the Zn-matrix.

The first aspect of this invention resides in a biocompatible and bioabsorbable composite material for full desorption *in vivo* in contact with a human or animal tissue, which comprises:

- a bioabsorbable Zn or Zn-alloy matrix forming a fine-grained bearing structure (preferably 90 to 99.9 % vol. of the composite material, the most preferably 96 to 99 % vol.), and
- 0.1 to 10 % vol. of a bioabsorbable component, relative to the volume of the composite material, desorbable *in vivo* in contact with a human or animal tissue, in the form of particles dispersed substantially homogenously in the matrix, wherein particle size of the component is 10 nm to 5 $\mu$m, preferably below 0.5 $\mu$m.

For the purpose of this invention the term 'fine-grained' refers to the grain size of 50 nm to 10 $\mu$m.

For the purpose of this invention the terms 'untreated zinc' and 'untreated zinc alloy' refer to the Zn or Zn alloy powder which is naturally passivated by ZnO films formed during production or storage.

The bioabsorbable Zn-alloy can be selected from the group comprising: preferably ZnMg, ZnCa, ZnCu, ZnMn, ZnLi, ZnFe, ZnSr, and other alloys of Zn (as a primary component by weight) and one or more additional elements such as Al, Ag, Ca, Co, Cu, Fe, Li, Mg, Mn, Ni, Ti, Sr, etc..

The bioabsorbable component is selected from the group comprising: metallic oxides, preferably bioabsorbable metallic oxides such as zinc oxide (ZnO), magnesium oxide (MgO), titanium dioxide ($TiO_2$), zirconium dioxide ($ZrO_2$), iron oxides, etc., rare earth element oxides such as yttrium oxide ($Y_2O_3$), strontium oxide (SrO), etc., calcium phosphate species such as hydroxyapatite, etc., pure metals and rare earth element such as Mg, Sr, Ag, etc., zinc hydrogen phosphate (HO4PZn), and other compounds, which consist of/comprises two or more of the following elements Zn, Ca, Mg, P, Y, Sr, O, H, Fe, etc.. The most preferably the bioabsorbable component is zinc oxide.

Particles of the bioabsorbable component are of discrete irregular platelets-like form, as they originate from films existing/formed on the surface of Zn or Zn alloy powder particles. During the extrusion process the films get fragmented and are introduced *in situ* into Zn or Zn alloy matrix grain structure.

The bioabsorbable composite material is a product of a powder consolidation of an untreated or surface modified Zn or Zn alloy powder.

According to the preferred embodiment, the bioabsorbable composite material according to the invention comprises 1 to 4% vol. of the bioabsorbable component.

The bioabsorbable composite material according to the invention is suitable for use as a biomedical implant such as an endovascular stent or an orthopaedic internal fixator. Further possible applications of the bioabsorbable composite material according the subject invention are e.g. bioresorbable dental membranes, functional biodegradable electronics and sensors.

The second aspect of this invention resides in a method of manufacturing of the bioabsorbable composite material wherein untreated or surface modified Zn or Zn alloy powder having a mean (median) particle size $d_{50}$ from 0.5 to 500 $\mu$m is extruded at temperature under 410 °C, wherein the reduction ratio during extrusion is 5:1 to 100:1, preferably 12:1.

According to the preferred embodiment the Zn or Zn alloy powder is extruded at a temperature 0 °C to 410 °C, preferably 0 °C to 100 °C, most preferably at a room temperature.

According to the further preferred embodiment a mean (median) particle size $d_{50}$ of Zn or Zn alloy powder is from 0.5 to 10 $\mu$m.

The extrusion process is preferably hydro-extrusion.

According to the yet another preferred embodiment, prior to the extrusion step the untreated or surface modified Zn or Zn alloy power is cold compacted by a cold isostatic pressing.

According to the yet another preferred embodiment after the extrusion step, the bioabsorbable composite material is machined or cut to a shape and dimensions of an implant.

The advantage of the method according to the invention is that it, in an elegant and simple manner, introduces *in situ* at low processing temperature rather small amount of nanometric second phase, which is very fine and dispersed evenly in principle within the matrix. On the top of that, it uses widely available powder component. That all results in low production costs. Moreover, the second phase is biocompatible and bioresorbable in principle, while it improves osseointegration and antibacterial properties at the same time. Refined Zn grain structure, fine nature of in-situ introduced second phase, its relatively low content and homogeneity across the volume oz Zn matrix yields superior mechanical performance, incl. fatigue and creep strengths and life, stability of the microstructure pinned by second phase, hence assures the microstructural stability upon processing, storage and service and its performance, homogenous corrosion at desired rates, non-toxicity, etc.

Zn dispersoids originate from passivating ZnO films, which exist on fine as-received e.g., as-atomized Zn powder. During the ME compaction ZnO films get fragmented and introduced in situ into refined Zn matrix grain structure. A primary role of ZnO disperoids is stabilization of a refined Zn grain structure during cold and hot working operations applied during Zn powder compaction and post-processing of Zn+ZnO. In this manner a post-deformation stability of the attractive mechanical properties of Zn+ZnO against age-hardening, which may take place during lagering and service at room temperature (RT) and the temperature of the human body (THB), is maintained. ZnO nanodispersoids located in the Zn matrix suppress a grain boundary rotation (GB rotation) and sliding creep deformation mechanisms, which in turn improves creep and fatigue performance. Furthermore, a small fraction of nanoscale ZnO dispersoids, which are homogenously dispersed in Zn matrix, pose no major deteriorating effect as stress concentrators and crack initiators on obtained mechanical properties.

As far as it concerns a biological effect of dispersoids, ZnO is a dominant product of Zn corrosion in the human body, which in addition to its nontoxicity, solubility and biocompatibility provides antibacterial properties, enhances the stem cell proliferation capacity, and provides anticancer properties.

The concept of in-situ stabilization by ZnO is universal for any Zn alloy matrix Use of suitable biocompatible and biodegradable Zn alloy, which meets requirements for application of biomedical devices such as ES and OIF, would result in a further increase of the mechanical strengths and fatigue strength limit. E.g., high cooling rates present during atomization process of Zn alloy powder (e.g., ZnMg with Mg < 1 wt.%) production and at the same time HE process realized at RT leads to formation of Zn solid solution structures and precipitation (e.g., $Zn_{11}Mg_2$) during fabrication, storage and service is suppressed. This, in addition to enhanced mechanical performance a post deformation stability of mechanical properties is assured, while a distinct loss of ductility is avoided.

Furthermore, various chemical, physical and mechanical methods can be used to replace or modify native passivating ZnO films present on the surface of as-received (as-atomised) fine Zn and Zn alloy powders by other substances e.g., pure metals, different metal and rare earth element oxides, calcium phosphate species, zinc hydrogen phosphate, etc for the purpose of enhanced corrosion and biological properties e.g., increased corrosion rate, more homogenous corrosion process, antibacterial feature, promoted osteointegration and osteoconduction potential, etc. of subsequent Zn based composites. Though, a surface modification of Zn and Zn alloy powder doesn't alter the above discussed concept of in-situ stabilization of refined Zn grain structure by ZnO nano dispersoids, which remain virtually the same.

**Brief description of the drawings**

Fig. 1a - represents SEM micrographs of as-atomized Zn powder in two scales.

Fig. 1b - represents the cumulative (Q3) and frequency distribution (dQ3) curves of the Zn powder particle size (x).

Fig. 2a and 2b - represent Electron Backscatter Diffraction (EBSD) data analysis of hydro extrusion Zn+ZnO for the transversal direction: IPF map shown in [001] crystal direction.

Fig. 2c - represents a grain size distribution chart with the insert of the corresponding pole figures.

Fig. 3a and 3b - represent EBSD data analysis of HE Zn+ZnO for the longitudinal direction: IPF map shown in [001] crystal direction. The arrow indicates the extrusion direction.

Fig. 4a - represents ADF STEM micrograph of HE Zn+ZnO.

Fig. 4b - represents the corresponding combined EDS map of Zn and O elements shown in transversal direction.

Fig. 5 - represents XRD patterns of HE Zn+ZnO and cast Zn reference.

Fig. 6 - represents ADF STEM micrograph of ZnO dispersoid and the corresponding combined EDS map of Zn and O elements in HE Zn+ZnO.

Fig. 7 - represents HAADF micrograph of ZnO dispersoid in HE Zn+ZnO with corresponding FFT. The marked area revealing the ZnO dispersoid [100] zone axis embedded within Zn matrix [210] zone axis.

Fig. 8 - displays the representative tensile stress-strain curves of Zn+ZnO in as-extruded state fabricated by hydroextrusion (HE Zn+ZnO) and after annealing at 100 °C for 24 h (HE+A Zn+ZnO).

Fig. 9 - displays the representative compressive stress-strain curves of HE Zn+ZnO obtained in the longitudinal and transversal direction in respect to the extrusion axes.
Fig. 10a - displays the apparent corrosion rate (CR)
Fig. 10b - displays graph representing a mass loss (ML) of HE Zn+ZnO and cast Zn as a function of the exposure time in DMEM.
Fig. 11a and 11b - display cell viability of L929 incubated with the undiluted (100%) and diluted (25 and 10%) extracts of the investigated HE Zn+ZnO and cast Zn reference specimens for 24 h and 72 h evaluated by an MTT assay.

**Detailed description**

Example 1

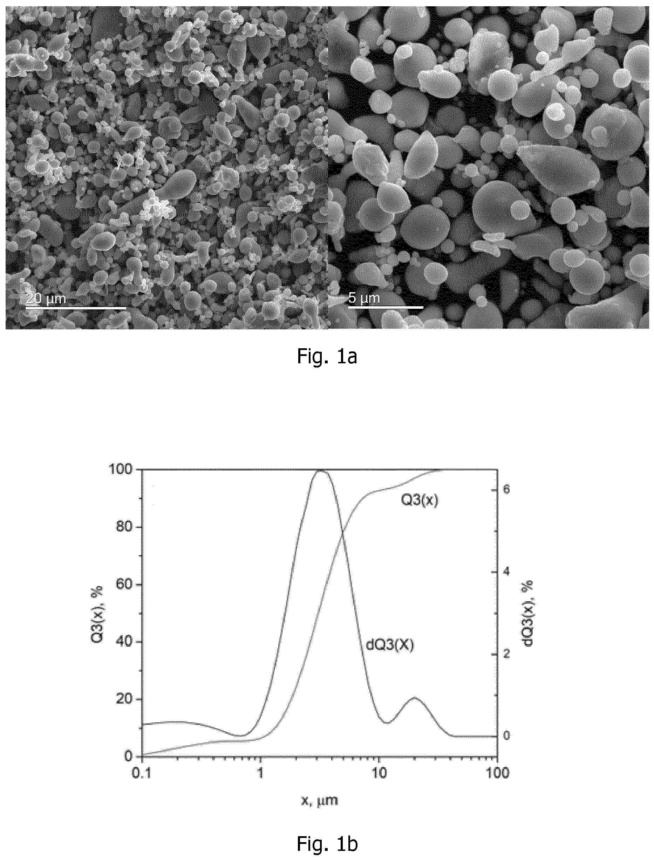

Even though the concept of in-situ stabilization by ZnO is universal for any Zn alloy matrix, for simplicity reason a proof of concept is demonstrated for a high purity Zn matrix i.e., a high purity Zn powder. We present mechanical testing, detailed microstructural characterization, confirmation of post-deformation stability, a corrosion behavior as well as biological in-vitro evaluation of novel bioabsorbable Zn+ZnO. Gas atomized 4 N Zn powder was used (**Fig. 1a**). Particle size distribution (PSD) was determined by a laser diffraction system by wet dispersion (Fritsch Analysette 22 MicroTec device). The $d_{10}$, $d_{50}$ and $d_{90}$ powder particle size values of 1.35, 3.21 and 7.45 $\mu$m were measured by PSD analyses, respectively (**Fig. 1b**). The Zn powder was precompacted by cold isostatic pressing (CIP) at a pressure of 200 MPa. CIPed blanks were inserted into the Cu sleeve and fully consolidated by HE, which was carried out at RT into rod bars with a diameter of 10 mm using a flat face die at a reduction ratio of ~12:1 and a ram speed of 15 mm.s$^{-1}$. For a sake of comparison, CIPed Zn powder blank was consolidated by CE at the identical conditions as those applied during HE.

As a reference the cast 4 N Zn material (supplied by Garda Alloys sro) was used. As-cast Zn was HE at the identical conditions as those applied for HE Zn+ZnO MMC. In order to study the structural stability and stability of the mechanical properties of HE Zn+ZnO and HE Zn a part of the as-extruded profiles were annealed at 100 °C for 24 h in argon. The materials in this patent application were labelled as shown in Table 1.

Table 1 - The designation of Zn+ZnO and Zn samples.

| feedstock material | condition | designation |
|---|---|---|
| **Zn powder** | hydroextruded | HE Zn+ZnO |
| | hydroextruded + annealed | HE+A Zn+ZnO |
| | conventionally extruded | CE Zn+ZnO |
| **Zn ingot** | cast | cast Zn |
| | cast + hydroextruded | HE Zn |
| | cast + hydroextruded + annealed | HE+A Zn |

Microstructural analysis

Density of the extruded Zn was determined by Archimedes' principle. The oxygen (O) content in HE Zn+ZnO was determined by hot gas fusion analysis (GFA; Bruker G8 Galileo OHN). The extruded bars were cut into small pieces, while sectioning was carried out in the transversal (T) and longitudinal (L) directions. The microstructure of the loose powder and HE Zn+ZnO was examined using a scanning electron microscope (SEM, FEI 3D Quanta 3D FEG) equipped with an electron back-scattering diffraction system (EBSD, TSL/EDAX Hikari) and EDAX OIM Analysis 8 software. The structure was studied by X-ray diffraction (XRD) using a D8 Advance (Bruker AXS) diffractometer (Cu Ka) in a parallel beam configuration. Direct microstructural investigations were performed with a probe-corrected FEI/Thermofisher Scientific Titan Themis 300 transmission electron microscope in scanning mode (STEM) at a 200 kV accelerating voltage equipped with an EDS system (Super-X).

*Results:*

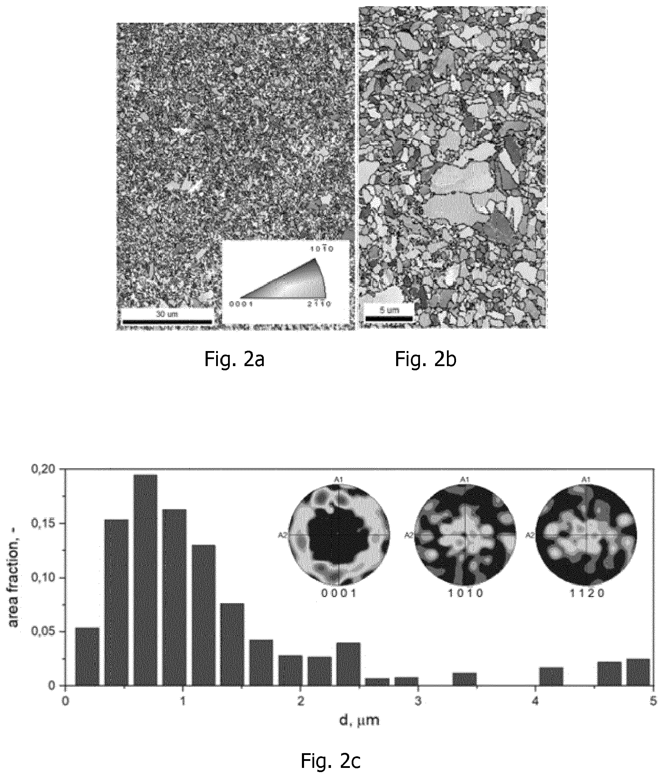

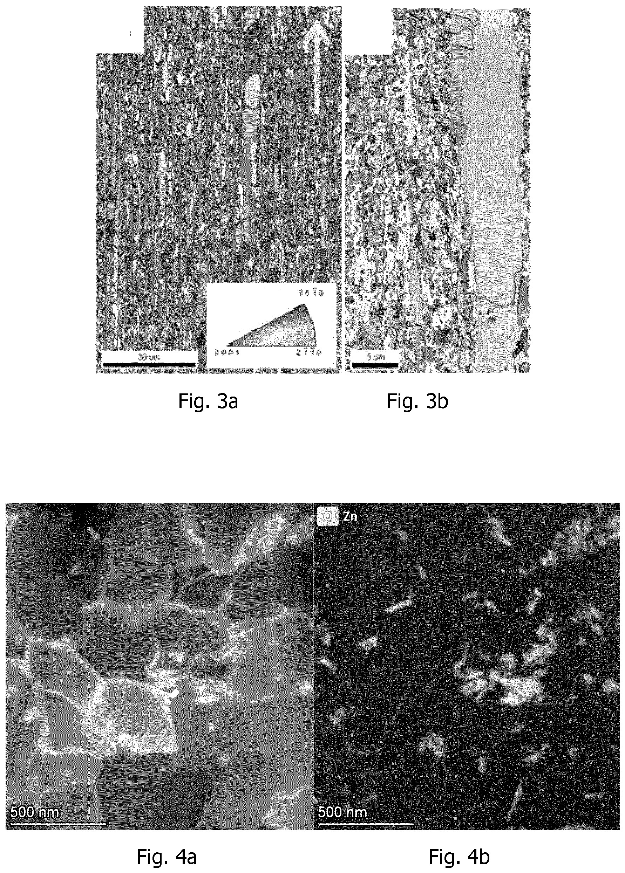

HE process resulted in almost full densification of Zn powder into a sound material. A measured density of HE Zn+ZnO was 7.014 g.cm$^{-3}$, which represented 98.7% of the theoretical density of Zn composite with the content of ZnO being 2.13 vol.%. The ZnO content was calculated from measured O ($0.33\pm0.03$ wt.%), whereas all O was attributed to the presence of ZnO component. **Figs. 2a, b** and **3a, b** show the grain orientation EBSD IPF maps of HE Zn+ZnO collected in the T and L directions, respectively. Hydrostatic condition and shearing introduced during HE resulted in a pronounced plastic deformation of Zn powder and refined Zn grain structure eventually. The microstructure in the T direction consisted of equiaxed Zn grains with an average grain size of $0.75\pm0.37$ $\mu$m and rather broad size distribution (**Figs. 2a, b, d**). As determined by the grain orientation spread (GOS) parameter in most of the grains were either fully recrystallized with well-developed HAGB and no obvious internal substructure or showed a little increased deformation. The low GOS parameter indicated a dynamic recovery, which took place during HE. Though, some namely larger grains contained a few low angle grain boundaries (LAGB). A few grains showed pronounced internal orientation spread. In the L direction Zn grain structure was characterized by a mixture of equiaxed grains and those elongated along the extrusion direction (**Fig. 3a, b**). The equiaxed grains tended to have a finer size. Conversely, elongated grains were mostly larger, had fragmented structure and were often part of the bands. Again, the GOS map proved that equiaxed fine grains were more uniform, while elongated coarser grains had larger GOS parameter mostly. On one hand, the existence of the bands might be attributed to shearing induced during HE, as often observed for highly deformed Zn-based materials. On the other hand, large elongated grains might stem from coarser Zn powder particles with an average size of ~20 $\mu$m, as seen in the bimodal PSD curve of as-atomized Zn powder (**Fig. 1b**). **Figs. 2a, 2b, 2c** and **3a, 3b** confirmed that HE Zn+ZnO was significantly textured with the basal planes being parallel with the extrusion direction, which is common for the extruded Zn. Although, the basal planes were inclined from the L direction to the T direction by a relatively large angle, with the maximum intensity at approximately 20°, which was attributed to the activity of the $2^{nd}$ order pyramidal slip.

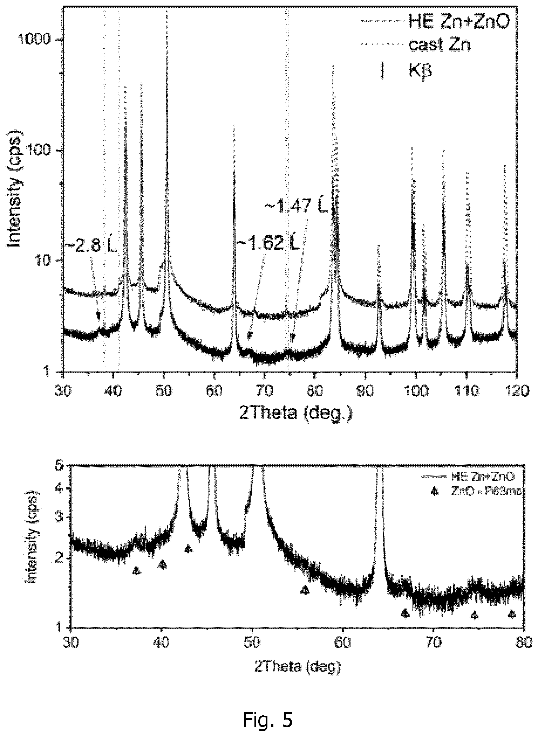

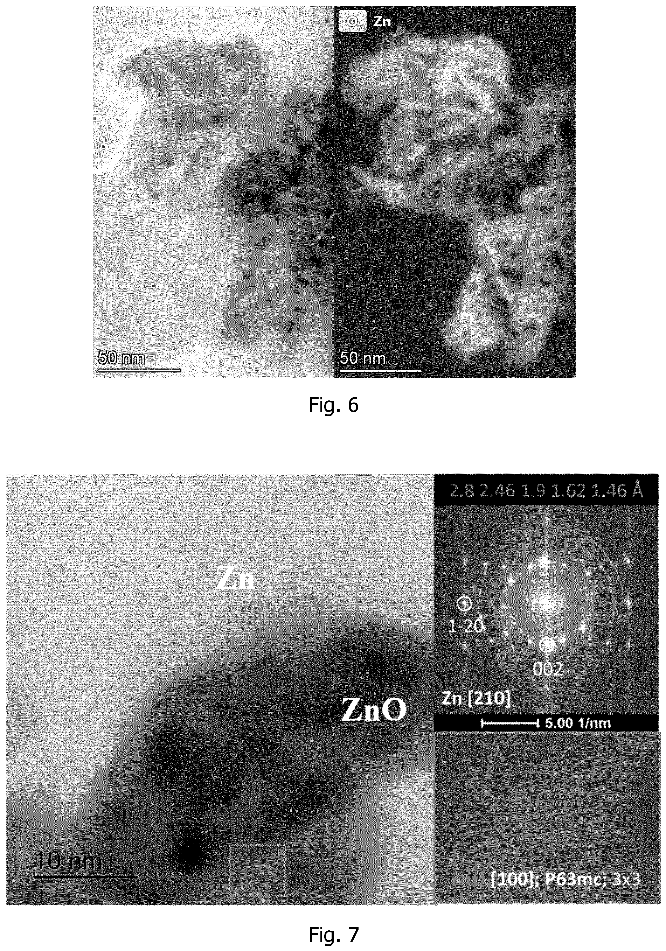

Shear introduced during HE deformed and fragmented ZnO passivation layers, which existed on as-atomized Zn powder. Fragmented ZnO platelets i.e., very fine dispersoids, mostly resided at HAGB (**Fig. 4a, 4b**). The presence of crystalline ZnO dispersoids was approved by XRD (**Fig. 5**), they possessed a wurtzite crystal structure with the $P6_3mc$ space group. As determined by GFA analyses the content of ZnO dispersoids in Zn matrix was rather small reaching 2.13 vol.%. The size of ZnO platelets, forming a porous, spongy-like structure was in general ~100 nm (**Fig. 6, 7**). From a microscopic point of view, the ZnO dispersoids were evenly dispersed in Zn matrix, while the estimated interparticle spacing was a couple of hundreds nm. The crystalline structure of the platelet agglomerates corresponded to the XRD results.

Owing to the particle-induced nucleation and Zener pinning mechanisms, the secondary-phase fine particles are known to accommodate local recrystallization, which yields intensive local grain refinement of metallic matrix in the vicinity of such particles. This appeared to be a case of HE Zn+ZnO, which showed fully recrystallized and fine Zn grains decorated with ZnO nanoscale dispersoids at HAGB. By ZnO dispersoids pinning Zener pressure was exerted on HAGB. As soon as Zener pressure reaches the driving pressure owing to the boundary curvature and the driving force for recrystallization, recrystallization and coarsening are suppressed. The dz value, at which the driving force for migrating GB is balanced by the pinning action of arbitrarily scattered spherical dispersoids with the average size c and the fraction f, can be estimated as follows:

$$d_Z = \frac{c}{6\,f}.$$

Assuming a migrating GB being anchored by ZnO dispersoids with ~2.13 vol.% population evenly dispersed in Zn matrix and having an average size of 100 nm the dz = ~0.78 $\mu$m is obtained. This value was in a good agreement with an average transversal grain size (0.75 $\mu$m) of HE Zn+ZnO determined by EBSD. It pointed to an effective stabilization of refined Zn grain structure by introduced ZnO dispersoids against recrystallization and grain growth during and after HE process. Thus, further refinement of Zn grain structure is possible by increasing f, what can be achieved practically by utilization of finer Zn feed-stock powder for Zn+ZnO production.

Mechanical testing

The mechanical testing was performed using Zn+ZnO and Zn materials using tensile specimens with a gauge diameter of 3 mm and a length of 21 mm, which were machined in parallel to the direction of extrusion. The tensile tests were performed at 23 °C (RT) using a Zwick Roell 1474 machine in accordance with the ASTM E8 standard. A cross-head speed was set up to 0.7 mm.min$^{-1}$, which represented an average strain rate ($\dot{\varepsilon}$) of $3\cdot10^{-4}$ and $6\cdot10^{-4}$ s$^{-1}$ below and above a yield point, respectively.

Additionally, compression testing was performed at RT using cylinders with a diameter of 3 mm and a height of 5 mm machined in the L and T directions. A cross-head speed during tests was set up to 0.5 mm·min$^{-1}$, which represented an average strain rate of $3\cdot10^{-3}$ and $6\cdot10^{-3}$ s$^{-1}$ below and above a yield point, respectively. Young's modulus (E) of HE Zn+ZnO and HE Zn was investigated through a three-point bending test using dynamic mechanical analysis (DMA, TA Instruments Q800) using specimens with the dimensions of 4·2·55 mm$^3$ machined in the L direction. The DMA experiment was performed at a preload force of 0.5 N, a frequency of 1 Hz, and a sinusoidal force amplitude of 7 N for 4 min at RT.

*Results:*

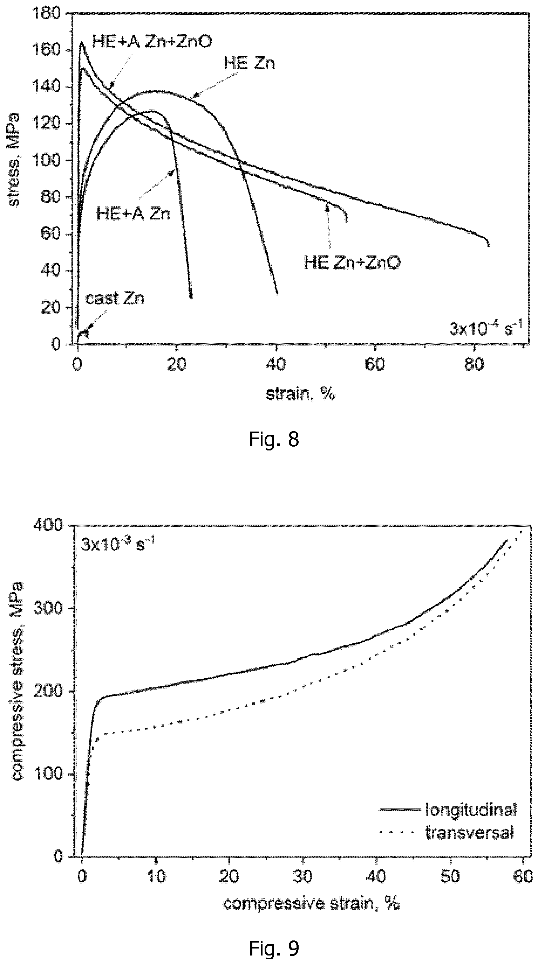

Application of HE was proven to enhance significantly the mechanical properties of the cast Zn-1Mg alloy to the best combination of strength and plasticity of ZnMg alloys. It was explained by an efficient grain refinement induced during low-temperature straining and partial suppression of DRX. In the present invention we confirmed that the poor mechanical properties, strengths, as well as A of cast Zn improved noticeably, more than by an order of magnitude, after the application of HE deformation (**Fig. 8, Tab. 2**)**.** HE accommodated substantial grain refinement and the area of GB increased markedly. GB worked as efficient obstacles, which impeded dislocation slip and promoted GB sliding, allowing for strengthening and greater plasticity at the same time. The mechanical properties of HE Zn+ZnO manufactured under the same conditions as HE Zn were superior to those of HE Zn counterpart. The stress-strain curve of HE Zn+ZnO differed markedly from the one seen for HE Zn (**Fig. 8**). HE Zn+ZnO featured a limited strain hardening ability in an elastic region, rather similar UTS and $YS_{0.2}$ values were confirmed, UTS was reached right after yielding at rather low strain value of 1%, followed by significant strain softening till fracture at the high values of A. A similar deformation behavior was found typical for near and submicrometre Zn-based materials, and other metals and MMC. It is rationalized by an existence of an inhomogeneous flow due to limited dislocation ability and dislocation-dislocation interaction within small grain interiors, where the mean free path of dislocation is governed mostly by HAGB. A plastic instability occurs as a consequence of a large surface area of HAGB with related dislocation sink effect and shortage of mobile dislocations.
While UTS of HE Zn+ZnO increased slightly by 8%, YS0.2 was boosted by 50% compared to the values determined for HE Zn counterpart. This could be rationalized by a positive effect of fine ZnO dispersoids. On one hand, ZnO positioned dominantly at HAGB didn't contribute to the Orowan strengthening mechanism markedly and they didn't act directly as obstacles to moving dislocations. On the other hand, ZnO dispersoids accommodated intensive grain refinement of Zn grain structure due to particle-induced nucleation and Zener pinning action, which resulted in an increase of GB mediated strengthening. In addition, ZnO presence at HAGB impeded mechanically GB sliding deformation mechanism, allowing for higher YS0.2. As reported for other metallic systems, an excellent tradeoff between strengths and A of HE Zn+ZnO can be attributed to the bimodal Zn grain size distribution too. Owing to a minor texture and the presence of some elongated Zn grains higher strength of HE Zn+ZnO was confirmed in the L rather than in the T direction (**Fig**. **9**). The efficiency of the HE deformation process realized at the hydrostatic and almost frictionless conditions was demonstrated for Zn+ZnO extruded at the same parameters but using CE set-up. First, HE yielded a significantly reduced breakthrough pressure of 890 MPa compared to the extremely high value of 1500 MPa determined during CE, which was at the limit of utilized laboratory CE set-up with high-pressure capacity. Second, in comparison to CE Zn+ZnO the UTS and $YS_{0.2}$ values of HE Zn+ZnO increased by ~25 and 40%, respectively.

**Table 2.** Ultimate tensile strength (UTS), 0.2% strain offset yield stress ($YS_{0.2}$), ductility (A), and Young's modulus (E) of Zn+ZnO in as-extruded state fabricated by hydroextrusion (HE Zn+ZnO) and after annealing at 100 °C for 24 h (HE+A Zn+ZnO). For comparison data on Zn+ZnO in as-extruded state fabricated by conventional extrusion (CE Zn+ZnO), cast Zn, Zn in as-extruded state (HE Zn) and after annealing at 100 °C for 24 h (HE+A Zn) is included.

| condition | UTS, MPa | YS$_{0.2}$, MPa | A, % | E, GPa |
|---|---|---|---|---|
| **HE Zn+ZnO** | 150 | 133 | 54 | 89.6 |
| **HE+A Zn+ZnO** | 164 | 154 | 83 | - |
| **CE Zn+ZnO** | 122 | 94 | 70 | |
| **cast Zn** | 8 | 6 | 2 | - |
| **HE Zn** | 139 | 67 | 44 | 85.7 |
| **HE+A Zn** | 130 | 60 | 25 | - |

On one hand, DRX accompanied by microstructure coarsening characteristically takes place in low-alloyed Zn. On the other hand, natural ageing processes active during storage and service in precipitation strengthened Zn alloys e.g., typically by $Zn_{11}Mg_2$ precipitates, lead to instability of the mechanical properties. In the absence of externally applied stress, a concept of stabilization by stable and fine dispersoids evenly distributed in the metallic matrix is effective up to high homologous temperatures of 0.93-0.99. An actual stabilization effect induced by ZnO dispersoids, which pinned Zn grain structure, was further demonstrated for HE Zn+ZnO annealed at 100 °C for 24 h. The testing temperature of 100 °C represented a homologous temperature of 0.54. The annealing at 100 °C for 24 h may be considered as an accelerated thermal treatment, which simulates long term service of biomedical device at THB. Annealing didn't lead to microstructural change and grain growth, which would be accompanied by a strength decline inevitably. Conversely, the strengths, as well as A improved slightly further for HE+A Zn+ZnO. Compared to HE Zn+ZnO the UTS, $YS_{0.2}$ and A increased by 9, 16 and 54% after annealing, respectively. This behavior contradicted with ZnO free HE+A Zn subjected to the same thermal treatment, where an obvious deterioration of the mechanical properties took place. Compared to as-extruded conditions UTS, $YS_{0.2}$ and A of annealed HE+A Zn decreased by 7, 10 and 43%, respectively. It's worth pointing out, that to our knowledge HE+A Zn+ZnO material in the present invention showed the highest UTS, $YS_{0.2}$ as well as A ever reported for pure Zn.

E of HE Zn+ZnO increased by 5% to 89.6 GPa compared to ZnO free counterpart material (**Tab. 2**), which may be explained (to some extent) by presence of ZnO dispersoids, with a higher E of 140 GPa than pure Zn matrix.

Corrosion testing

The degradation behavior of HE Zn+ZnO and cast Zn was accessed by static immersion and immersion tests. For that, multiple cylindrical samples of each material were machined, each one having $4\pm0.03$ mm in a diameter and $8\pm0.09$ mm in a length.

*Immersion tests*

The specimens underwent ultrasonic cleaning in isopropyl alcohol, were dried with a jet air, sterilized with UV-C and were then immersed separately in the sterilized bottles having 50.5 mL (using a ratio of 40 ml·cm$^{-2}$) of high-glucose Dulbecco's Modified Eagle Medium (DMEM; Biosera) supplemented with 5% Fetal Bovine Serum (FBS Superior, Sigma-Aldrich, Germany) for up to 21 days or until a stable mass loss (ML) was reached. The bottles were kept in a controlled atmosphere enriched with 5% $CO_2$ at 37 °C for mimetic body-environment conditions. The pH index of each system was monitored every 2 days using a pH meter (PC 7+ device) with an accuracy of $\pm0.1$. ML measurements were applied according to the standard practice for the laboratory immersion corrosion testing of metals (ASTM G31-72), to evaluate the corrosion rate (CR) of as-extruded Zn+ZnO and as-cast Zn. After the specified time of immersion, the cylinders were soaked in chemical cleaning solution containing 200 g·L$^{-1}$ $Cr_2O_3$ for 2 minutes to remove the corrosion products on the sample surfaces, then rinsed in distillate water, and dried to be weighted. ML was calculated as follows:

$$ML = \frac{m_i - m_f}{A}$$

where $m_i$ is the initial weight of the sample before immersion, $m_f$ is the final weight of each sample after immersion, and A is the sample's surface area. CR was calculated as follows:

$$CR = K\frac{(ML)}{D \cdot t}$$

where K is a constant equal to 87.4, D is the density of the tested material and t is the immersion time. Since the mass loss measurements give only the apparent corrosion rate, different time intervals were applied (2, 4, 7, or 14 days) for accessing the degradation rate at the early stages of the corrosion.

*Results:*

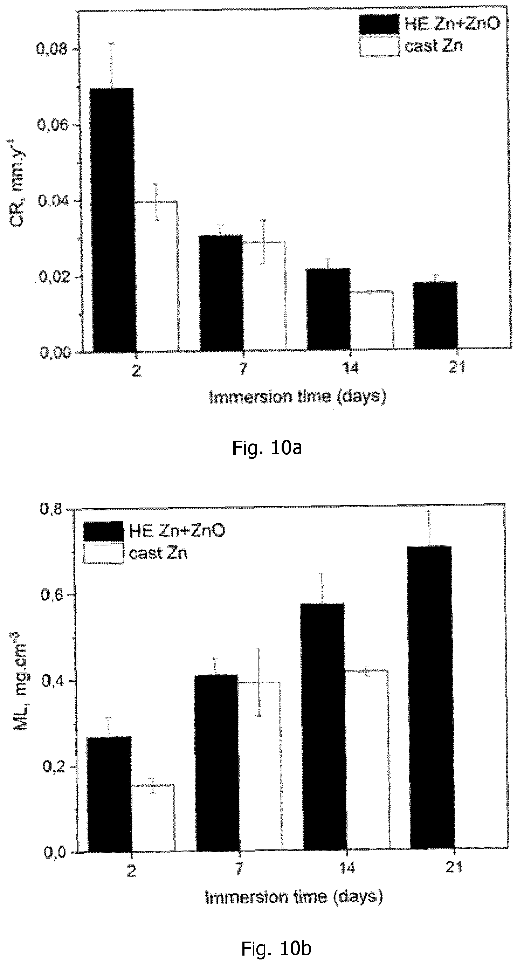

The apparent CR after 2 weeks of immersion in DMEM calculated for each group of materials was $0.021\pm0.003$ and $0.015\pm0.001$ mm·y$^{-1}$ for HE Zn+ZnO and cast Zn, respectively. The values agreed with those one reported in the literature for pure Zn and its alloys. These results were also comparable with the reported CR between 0.02 mm·y$^{-1}$ to 0.03 mm·y$^{-1}$ determined during in-vivo studies of Zn implanted in rat's abdominal aorta. **Figs. 10a and 10b** shows the evolution of the apparent CR and ML for HE Zn+ZnO and cast Zn as a function of the immersion period. Throughout the experiment, the monitored variations to pH were minimal, fluctuating from 7.4 to 7.7. It can be seen that, at the early stages of corrosion, CR after 48 h of immersion was higher for HE Zn+ZnO compared to cast Zn reference, but this difference tended to diminish for the longer immersion times. After 3 weeks, the apparent CR calculated for HE Zn+ZnO dropped down to $0.018\pm0.002$ mm·y$^{-1}$, which complied with the standard rate defined for degradable ES materials i.e., 0.02 mm·y$^{-1}$.

*In-vitro* biological evaluation

*Cytotoxicity MTT assay*

For indirect contact cytotoxicity assays, the extraction of HE Zn+ZnO and cast Zn specimens was executed in a DMEM + 10% FCS medium at a ratio of 0.2 g·ml$^{-1}$ at 37 °C under continuous shaking of 120 rpm for 24 h in sterile containers according to the ISO 10993-12 guidelines. The cylindrical samples with a diameter of 5 mm and a length of 9 mm were autoclave sterilized for 60 min. The samples had the same surface characteristics as those used for corrosion immersion testing experiments. A 100, 25 and 10% extracts were used for all in-vitro experiments. The L929 at a cell density of 10000 cells·well$^{-1}$ were seeded into 96-well plates and incubated at 37 °C, in 5% $CO_2$ for 24 h. The following day, the extracts from the tested samples were added to the monolayers of the cells and incubated for two different exposure durations, 24 and 72 h, at 37 °C, in 5% $CO_2$. According to standard ISO 10993-5, a decrease greater than 30% was considered as the limiting value between nontoxic and toxic behavior.

*Results:*

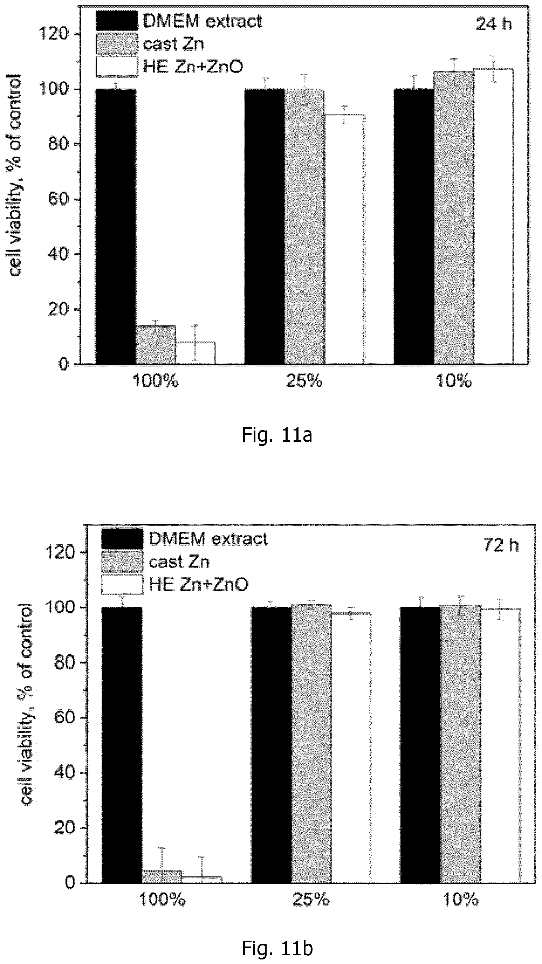

The potential cytotoxic response of the extraction media of HE Zn+ZnO and the cast Zn materials to the L929 cells are shown in **Figs. 11a and 11b.** Both materials exhibited rather comparable behavior. The undiluted extracts of both materials imposed a strong toxic effect to the L929 cells upon 24 and 72 h treatments. Conversely, dilution of extracts down to 25 and 10% resulted in non-cytotoxic behavior according to the ISO 10993-5 criteria i.e., the normalized cell viability was way over 70%. Such findings on a toxicity level of Zn based materials to sensitive L929 cells were in a line with other studies and it was rationalized on a basis of the highest safe Zn ion concentration (80 $\mu$M) in the DMEM extract. It's accepted that high amount of Zn ions causes a toxic effect. It was shown that an extensive cytotoxic behavior of Zn-0.8Mg material diminished abruptly when Zn ion concentration decreased down to 44 $\mu$M as experimentally confirmed for the 25% diluted DMEM+5% FBS extract. It was assumed, that local Zn concentrations limits in the vicinity of Zn implants covered with corrosion products in the surrounding fluid are well below the safe values, proven by successful in-vivo studies.

Conclusions

Novel biomedical Zn+ZnO MMC was manufactured by PM approach using HE compaction of fine gas-atomized high purity Zn powder feedstock. The microstructure, mechanical properties, post-production stability, corrosion behavior and in-vitro biological evaluation were pursued. The results were compared with those obtained for the cast Zn reference. The following conclusions were drawn:

- HE resulted in the sound composite material characterized by a refined Zn matrix grain structure with the average grain size of 0.75 $\mu$m. Zn grain structure was formed dominantly by the fine recrystallized equiaxed grains and coarser fragmented grains elongated along the extrusion direction.
- The structure was significantly textured with the basal planes parallel and inclined by ~20° with the extrusion direction, which was attributed to the activity of the 2nd order pyramidal slip.
- 2.13 vol.% of ZnO dispersoids with the size of ~100 nm, which stemmed from passivating ZnO films on as-atomized Zn powder, were evenly dispersed within Zn grain structure and resided dominantly at Zn HAGB.
- ZnO dispersoids, which effectively refined Zn grain structure during HE, grain pinned Zn structure during post annealing realized at 100 °C for 24 h by Zener stabilization and thus prevented it from unwanted microstructural changes e.g., coarsening.
- Annealed Zn+ZnO showed the tensile mechanical properties (UTS=164 MPa, YS$_{0.2}$=154 MPa and A=83%), which were the values superior to all those reported for pure Zn in the literature.
- After 3 weeks of exposure to DMEM, a corrosion process in HE Zn+ZnO with apparent CR=0.018 mm·y$^{-1}$ was slightly faster as opposed to the cast Zn.
- There was no change in the electrochemical reactions of HE Zn+ZnO and the cast Zn. During the first few days HE Zn+ZnO had a faster rate of charge exchange than the cast Zn, while after a week's exposure, the response of both materials was very similar.
- The *in-vitro* cytotoxicity tests with material extracts and the L929 cells resulted in comparable behavior of HE Zn+ZnO and the cast Zn. Undiluted extracts imposed a strong toxic effect, while the 25% diluted extracts resulted in non-cytotoxic behavior.

Example 2

Table 3

| Sample # | Zinc matrix | grain size of bearing structure | Component | % vol. of component | Component's particle size |
|---|---|---|---|---|---|
| 1 | Zn | 1.5 $\mu$m | ZnO | 0.9 | ~100 nm |
| 2 | Zn | 0.55 $\mu$m | ZnO | 2.9 | ~100 nm |
| 3 | Zn | 0.42 $\mu$m | ZnO | 3.5 | ~100 nm |
| 4 | Zn | 0.36 $\mu$m | ZnO | 3.9 | ~100 nm |
| 5 | Zn | 0.31 $\mu$m | ZnO | 5.1 | ~100 nm |

The composite materials shown in **Tab. 3** were prepared by the method described in Example 1. but using the different feedstock Zn powders with d50=10.5 $\mu$m (#1), d50=2.1 $\mu$m (#2), d50=1.5 $\mu$m (#3), d50=0.9 $\mu$m (#4), and d50=0.6 $\mu$m (#5). The composites were subjected to similar characterization and testing as in Example 1 with the following results:

- The strength (YS0.2 and UTS) of the Zn+ZnO composites increased with the decrease of the grain size. The ductility of of the Zn+ZnO composites decreased with the decrease of the grain size but this decrease was rather minor.
- Regardless of the amount of induced ZnO component, thermal stability of refined Zn grain structure attained by pinning action of Zn component was assured.
- The corrosion properties were found independent of the Zn grain size and ZnO component content.

Example 3

Table 4

| Sample # | Zinc-alloy matrix | grain size of bearing structure | Component | % vol. of component | Component's particle size |
|---|---|---|---|---|---|
| 1 | Zn-0.8Mg | 0.75 | ZnO | 2.1 | ~100 nm |
| 2 | Zn-0.8Mg | 0.54 | ZnO | 3.0 | ~100 nm |
| 1 | Zn-1Ca | 0.75 | ZnO | 2.1 | ~100 nm |
| 2 | Zn-1Ca-0.8Mg | 0.75 | ZnO | 2.1 | ~100 nm |

The composite materials shown in **Tab. 4** were prepared by the method described in Example 1 but using the different feedstock Zn-0.8Mg, Zn-1Ca and Zn-1Ca-0.8Mg alloyed powders with d50=3.5 $\mu$m (#1) and d50=2.1 $\mu$m (#2). The composites were subjected to similar characterization and testing as in Example 1 with the following results:

- Owing to fast-cooling rates present during gas atomization processed used to produce both fine nature Zn alloyed powders, Mg and Ca elements were found present mostly in a solid solution of Zn cell structure. Such structure was preserved during HE process realized at RT and no precipitation of e.g., $Zn_{11}Mg_2$ took place upon HE and long term storage at the temperature of a human body. At the same time ZnO component effectively stabilized the Zn grain structure and no grain growth took place.
- The strength (YS0.2 and UTS) of the ZnMg0.8+ZnO composites increased with the decrease of the grain size.
- Significant increase of the strength (YS0.2 and UTS) was confirmed compared to Zn+ZnO counterparts with the same Zn grain size.
- The ductility of the ZnMg0.8+ZnO composites was sufficiently high for the application of ES and OIF application.

Example 4

Table 5

| Sample # | Zinc-alloy matrix | grain size of bearing structure | Component | % vol. of component | Component's particle size |
|---|---|---|---|---|---|
| 1 | Zn | 0.75 | MgO | 1.5 | ~100 nm |
| 2 | Zn | 0.75 | HO4PZn | 3.0 | ~100 nm |

The composite materials shown in **Tab. 5** were prepared by the method described in Example 1. but using the different feedstock surface modified Zn powders with d50=3.5 $\mu$m and MgO (#1) and HO4PZn (#2) component. The composites were subjected to similar characterization and testing as in Example 1 with the following results:

- Fracture of MgO and HO4PZn layers takes place during HE process to similar matter as the once determined for untreated Zn powder and native passivating ZnO films.
- MgO and HO4PZn dispersoids provided similar stabilization effect to Zn grain structure as for untreated Zn powder and native passivating ZnO films and hence similar mechanical properties were obtained.

**References**

[1] J.A. Finegold, P. Asaria, D.P. Francis, Int. J. Cardiol. 168 (2013) 934e945.
[2] S. Cook, P. Wenaweser, M. Togni, M. Billinger, C. Morger, C. Seiler, R. Vogel, O. Hess, B. Meier, S. Windecker, Circulation 115 (2007) 2426e2434.
[3] J. Venezuela, M.S. Dargusch, Acta Biomater. 87 (2019) 1.
[4] C.J. Frederickson, J.Y. Koh, A.I. Bush, Nat. Rev. Neurosci. 6 (2005) 449.
[5] P.K. Bowen, J. Drelich, J. Goldman, Adv. Mater. 25 (2013) 2577.
[6] H. Jin, S. Zhao, R. Guillory, P.K. Bowen, Z.Y. Yin, A. Griebel, J. Schaffer, E.J. Earley, J. Goldman, J.W. Drelich, Mater. Sci. Eng. C 84 (2018) 67.
[7] H. Yang, B. Jia, Z. Zhang, X. Qu, G. Li, W. Lin, D. Zhu, K. Dai, Y. Zheng, Nat. Commun. 11:401 (2020) 1-16.
[8] K. Piela, L. Blaz, W. Bochniak, P. Ostachowski, M. Lagoda, P. Zabinski, M. Jaskowski, M. Kiper, A. Polkowska, J. Alloys Compd. 810 (2019) 151883.

**Claims**

1.  A biocompatible and bioabsorbable composite material for full absorption *in vivo* in contact with a human or animal tissue, **characterized in that**, it comprises:

    - a bioabsorbable zinc or zinc-alloy matrix forming a fine-grained bearing structure and
    - 0.1 to 10 % vol. of a bioabsorbable component, relative to the volume of the composite material, absorbable *in vivo* in contact with a human or animal tissue, in the form of particles dispersed substantially homogenously in the matrix, wherein particle size is 10 nm to 5 $\mu$m, preferably below 0.5 $\mu$m.

2.  The composite material according to the claim 1, **characterized in that**, the bioabsorbable zinc-alloy is selected from the group comprising: ZnMg, ZnCa, ZnCu, ZnMn, ZnLi, ZnFe, ZnSr or Zn-alloy comprising Zn as a primary component by weight, and one or more additional elements selected from the group comprising: Al, Ag, Ca, Co, Cu, Fe, Li, Mg, Mn, Ni, Ti, Sr.

3.  The composite material according to the claim 1, **characterized in that**, the bioabsorbable component is selected from the group comprising: metallic oxides such as zinc oxide (ZnO), magnesium oxide (MgO), titanium dioxide ($TiO_2$), zirconium dioxide ($ZrO_2$), iron oxides; rare earth element oxides such as yttrium oxide ($Y_2O_3$), strontium oxide (SrO); calcium phosphate species such as hydroxyapatite; pure metals and rare earth elements such as Mg, Sr, Ag; zinc hydrogen phosphate (HO4PZn) and other compounds, which comprise two or more of the following elements: Zn, Ca, Mg, P, Y, Sr, O, H, Fe; the most preferably zinc oxide.

4. The composite material according to any of the preceding claims, **characterized in that** the particles of the bioabsorbable component are of discrete irregular platelets-like form.

5. The composite material according to any of the preceding claims, **characterized in that**, the composite material is a product of a powder consolidation of an untreated or surface modified zinc or zinc alloy powder.

6. The composite material according to any of the preceding claims, **characterized in that**, it comprises 1 to 4% vol. of the component.

7. Endovascular stent or an orthopaedic internal fixator consisting of the composite material according to any of the preceding claims.

8. A process of manufacturing of the composite material according to any of the proceeding claims, wherein untreated or surface modified zinc or zinc alloy powder having a mean (median) particle size $d_{50}$ from 0.5 to 500 $\mu$m is extruded at temperature under 410 °C, wherein the reduction ratio during extrusion is 5:1 to 100:1, preferably 12:1.

9. The process according to the claim 8, wherein the powder is extruded at a temperature 0 °C to 410 °C, preferably at a temperature 4 °C to 100°C, the most preferably at room temperature.

10. The process according to any of the claims 8 to 9, wherein the extrusion process is hydro-extrusion process.

11. The process according to any of the claims 8 to 10, wherein before the extrusion step the untreated or surface modified zinc or zinc alloy power is cold compacted by a cold isostatic pressing.

12. The process according to any of the claims 8 to 11, wherein after the extrusion step, the composite material is machined or cut to a shape and dimensions of an implant.

Fig. 1a

Fig. 1b

Fig. 2a                    Fig. 2b

Fig. 2c

Fig. 3a                    Fig. 3b

Fig. 4a                    Fig. 4b

Fig. 5

Fig. 6

Fig. 7

Fig. 8

Fig. 9

Fig. 10a

Fig. 10b

Fig. 11a

Fig. 11b

Europäisches
Patentamt

European
Patent Office

Office européen
des brevets

**EUROPEAN SEARCH REPORT**

**Application Number**

EP 22 19 1338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/353835 A1 (GUAN ZEYI [US] ET AL) 18 November 2021 (2021-11-18) <br> * paragraph [0028] - paragraph [0035] * <br> * paragraph [0037] * <br> * paragraph [0042] * <br> * paragraph [0046] - paragraph [0052]; claims; examples * <br> ----- | 1-12 | INV. <br> A61L27/04 <br> A61L31/02 <br> B21C23/00 <br> C22C18/00 <br> B22F1/052 <br> B22F3/04 <br> B22F3/20 |
| A | WO 2021/243398 A1 (UNIV DEAKIN [AU]) 9 December 2021 (2021-12-09) <br> * the whole document * <br> ----- | 1-12 | C22C1/04 <br> C22C1/05 <br> C22C1/10 <br> C22C32/00 |
| A | JARZEBSKA A ET AL: "A new approach to plastic deformation of biodegradable zinc alloy with magnesium and its effect on microstructure and mechanical properties", MATERIALS LETTERS, vol. 211, 15 January 2018 (2018-01-15), pages 58-61, XP085274102, ISSN: 0167-577X, DOI: 10.1016/J.MATLET.2017.09.090 <br> * the whole document * <br> ----- | 1-12 | |
| | | | TECHNICAL FIELDS SEARCHED (IPC) |
| A | MOSTAED EHSAN ET AL: "Zinc-based alloys for degradable vascular stent applications", ACTA BIOMATERIALIA, ELSEVIER, AMSTERDAM, NL, vol. 71, 10 March 2018 (2018-03-10), pages 1-23, XP085377867, ISSN: 1742-7061, DOI: 10.1016/J.ACTBIO.2018.03.005 <br> * the whole document * <br> ----- | 1-12 | A61L <br> B21L <br> B21C <br> C22C <br> B22F |

-/--

The present search report has been drawn up for all claims

| Place of search | Date of completion of the search | Examiner |
|---|---|---|
| The Hague | 30 January 2023 | Derrien, Anne-Cécile |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 22 19 1338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MCCARTHY W. H. ET AL: "Compression Strength of Zinc/Zinc Oxide Composites at Temperatures above the Melting Point of Zinc", METAL SCIENCE JOURNAL, vol. 4, no. 1, 18 January 1970 (1970-01-18), pages 74-77, XP093016408, ISSN: 0026-0681, DOI: 10.1179/030634570790444086 Retrieved from the Internet: URL:http://dx.doi.org/10.1179/030634570790444086> * the whole document * ----- | 1-12 | |

TECHNICAL FIELDS
SEARCHED (IPC)

The present search report has been drawn up for all claims

| Place of search | Date of completion of the search | Examiner |
|---|---|---|
| The Hague | 30 January 2023 | Derrien, Anne-Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 2 of 2**

## ANNEX TO THE EUROPEAN SEARCH REPORT
## ON EUROPEAN PATENT APPLICATION NO.

EP 22 19 1338

30-01-2023

| Patent document cited in search report | | Publication date | Patent family member(s) | | Publication date |
|---|---|---|---|---|---|
| US 2021353835 | A1 | 18-11-2021 | US | 2021353835 A1 | 18-11-2021 |
| | | | WO | 2020028643 A1 | 06-02-2020 |
| WO 2021243398 | A1 | 09-12-2021 | NONE | | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 52540353835 A1

### Non-patent literature cited in the description

- J.A. FINEGOLD ; P. ASARIA ; D.P. FRANCIS. *Int. J. Cardiol.,* 2013, vol. 168, 934e945
- S. COOK ; P. WENAWESER ; M. TOGNI ; M. BILLINGER ; C. MORGER ; C. SEILER ; R. VOGEL ; O. HESS ; B. MEIER ; S. WINDECKER. *Circulation,* 2007, vol. 115, 2426-2434
- J. VENEZUELA ; M.S. DARGUSCH. *Acta Biomater.,* 2019, vol. 87, 1
- C.J. FREDERICKSON ; J.Y. KOH ; A.I. BUSH. *Nat. Rev. Neurosci.,* 2005, vol. 6, 449
- P.K. BOWEN ; J. DRELICH ; J. GOLDMAN. *Adv. Mater.,* 2013, vol. 25, 2577
- H. JIN ; S. ZHAO ; R. GUILLORY ; P.K. BOWEN ; Z.Y. YIN ; A. GRIEBE ; J. SCHAFFER ; E.J. EARLEY ; J. GOLDMAN ; J.W. DRELICH. *Mater. Sci. Eng. C,* 2018, vol. 84, 67
- H. YANG ; B. JIA ; Z. ZHANG ; X. QU ; G. LI ; W. LIN ; D. ZHU ; K. DAI ; Y. ZHENG. *Nat. Commun.,* 2020, vol. 11 (401), 1-16
- K. PIELA ; L. BLAZ ; W. BOCHNIAK ; P. OSTACHOWSKI ; M. LAGODA ; P. ZABINSKI ; M. JASKOWSKI ; M. KIPER ; A. POLKOWSKA. *J. Alloys Compd.,* 2019, vol. 810, 151883